# EUROPEAN PATENT APPLICATION

(11) **EP 4 020 492 A1**
(43) Date of publication of application: **29.06.2022**
(21) Application number: 20217393.6
(22) Date of filing: 28.12.2020
(51) Int. Cl.: G16H 40/20, G16H 50/20, G16H 30/40

(54) **METHOD AND SYSTEM FOR AUTOMATICALLY DETERMINING (ALERTABLE) CHANGES OF A CONDITION OF A PATIENT**

(71) Applicant: Siemens Healthcare GmbH, 91052 Erlangen (DE)
(72) Inventor: HOLZER, Philipp, 08536 Plainsboro (US); CONJETI, Sailesh, 91056 Erlangen (DE)

(57) **Abstract**

The invention describes a method for automatically determining changes (C) of a condition of a patient (P), comprising the steps:
- receiving at least a first image (I1) and a second image (I2), both showing the same predefined anatomical region (TH) of the patient (P), wherein the images (I1, I2) have been recorded during different examinations of the patient (P),
- examining the images (I1, I2) for abnormalities (A) of a predetermined type, and in the case an abnormality (A) is found in at least one of the images (I1, I2):
- determining changes (C) of an abnormality (A) between the first image (I1) and the second image (I2),
- assessing the determined changes (C),
- determining whether the assessed changes (C) exceed a predefined threshold (T) by using a decision algorithm (D),
- in the case the assessed changes (C) exceed the predefined threshold (T): triggering an alert notification (N) and/or change a worklist prioritization concerning the respective patient (P).

The invention further describes a related determination system, a related client device and a related method performed with the determination system and the client device.

## Description

The invention describes a method and a system for automatically determining (alertable) changes of a condition of a patient, especially for generating or organizing a medical reading worklist, preferably for longitudinal (over two or more time points) triaging of chest x-rays.

Concerning dangerous courses of diseases, there are cases where a patient has a disposition of a certain disease, but there is no immediate danger. However, once there starts a rapid progression of the disease, there is an urge for treatment to prevent a fatal result. In the case there are many patients with such disposition, there may be the need of longitudinal triaging by prioritizing patients concerning this progression.

For example, a pneumothorax is a potentially life-threatening condition. When detected on a radiological exam such as x-ray or CT, it needs immediate patient management. It is very important to prioritize reading of those cases with a suspected pneumothorax. To this end, some solutions are available. These solutions are triggered once the image has been acquired and in the event of a found pneumothorax the corresponding mages are flagged and prioritized in the reading worklist. Hence the radiologist is alerted to these cases.

However, flagging every pneumothorax could potentially defeat its own purpose as this could mean that a considerable proportion of the worklist is prioritized and thereby eclipsing the urgent cases amongst stable cases. A significant proportion of chest x-rays are follow-up scans to monitor the progression of an already treated pneumothorax (e.g. with a chest tube). In these cases, a stable pneumothorax would not be considered an acute finding, were the pneumothorax to worsen however despite the chest tube, it would require immediate attention.

Triaging solutions for pneumothoraces are known. There is however no publicly disclosed solution to assess the context of the imaged pneumothorax and hence its criticality.

It is the object of the present invention to improve the known methods and systems to facilitate an improvement in determining changes of conditions of patients, especially concerning warning or triaging procedures.

This object is achieved by a method according to claim 1, a system according to claim 10, a client device according to claim 12 and a method according to claim 13.

In the following, the solution according to the invention is described with respect to the claimed systems as well as with respect to the claimed methods. Features, advantages or alternative embodiments herein can be assigned to the other corresponding claimed objects and vice versa. In other words, the systems can be improved with features described or claimed in the context of the corresponding method. In this case, the functional features of the methods are embodied by objective units of the systems.

A method according to the invention for automatically determining (alertable) changes of a condition of a patient, especially generating or organizing a medical reading worklist, preferably for longitudinal triaging of chest x-rays, comprises the following steps:
- Receiving at least a first image and a second image, both showing the same predefined anatomical region of the patient, wherein the images have been recorded during different examinations of the patient.

It is clear that both images should show the same region of interest, e.g. the same organ, since this region is examined for changes. It is also clear that the images should be recorded during different examinations of the patient, since a longitudinal examination is intended, i.e. an examination over two or more time points, e.g. at different hours, days, weeks or months, possibly at a different (physical) location. The more images are processed, the more timepoints are available. Any further image can be processed with this method with respect to the temporally adjacent image. Although it is preferred that both images are of the same type, e.g. both images are X-ray images or CT-images (CT: computer tomography), this is not in every case necessary. Instead of the term "image" also the term "medical image" can be used. For example, the image can be an object according to the DICOM (acronym for "Digital Imaging and Communications in Medicine") standard.
- examining the images for abnormalities of a predetermined type.

An abnormality is a pathological finding in an image, e.g. a radiographic finding. This is well known for medical examinations. The step is preferably accomplished by using a (deep) machine learning algorithm, that is trained for finding abnormalities in medical images. Such algorithms and their training are known in the art. It should be noted that it is not necessary to examine both images at the same time. It is also possible to examine the first image (a newer image than the second image) first and in the case an abnormality is found, the second image is received and examined. Thus, time can be spared while examining a large number of patients, by examining both images only in the case, where the newer image shows an abnormality. This preferred procedure is explained more accurately in the following.

In the case an abnormality is found in at least one of the images, the following steps are performed. In the case there is no abnormality found, the procedure could be started again in order to be performed with another patient or a newer image of the current patient. The steps in the "positive" case are:
- determining changes of the abnormality between the first and the second image (comparing the first image with the second image).

These changes could be changes of dimensions, e.g. the progression of the collapse of the lung concerning a pneumothorax or the expansion of a haemorrhage. For example, dimensional changes could be computed with area-comparison or apex to cupola distance comparison. However, changes could also be changes of opacity, texture and/or shape of an anatomical region. Concerning digital images, a "change" is a change in digital image information of the predefined region. Since the image information is usually the grayscale value of the pixels of the image, the change is the change of grayscale and/or size and/or form of image-elements in the predefined region.

It should be noted that using two images is the minimum to perform the method. The method can also be performed with three or more images recorded at different time-points, wherein changes should be determined in a timely order of the images such that the changes from time-point to time-point are determined for time-sequential time-points.
- assessing the determined changes.

To enable an automatic processing, the changes are brought in a condition where they can be numerically evaluated. This can e.g. happen by measuring the dimensional change (e.g. a pneumothorax could be measured following the American guidelines or the British guidelines, see e.g. Takaki Akamine "Interpleural distance predicts persistent air leak after initial primary spontaneous pneumothorax", J Thorac Dis. 2020 May; 12(5): 2228-2235), or by measuring the change in opacity, texture and/or shape. In a simple embodiment, the changes could be comparable numerical values.

It is preferred to register the images, however, registering is an optional step. Registration is advantageous, if the change comparison is at the level of a region of interest and could help with ensuring consistency in size measurements.

Since a "change" should be a numeric dataset, it could also be designated as "comparison value".
- determining whether the assessed changes exceed a predefined threshold by using a decision algorithm.

The threshold should be defined such that an actionable change that is complying with Standard of Care (or Clinical Standard Operating Procedures) is indicated. The Standard of Care as well as an actionable change is well known in the art concerning a predefined disease. It should be noted that an actionable change is not any change per say, but only ones that trigger changes to patient management. It should also be noted that the method according to the invention is not anticipating the examination of a physician, but may use the thresholds defined for actionable changes.

For automatically determining changes, said decision algorithm is used, that is designed to calculate whether changes, e.g. in the spatial extent (dimensions) and/or density, texture, shape of the abnormality manifest as an actionable change that is complying with Standard of care. This decision algorithm is necessary. It can be designed such that it is calculating whether a numerical value of a change exceeds a threshold in a very simple embodiment. However, it also could be a machine learning (especially deep learning) algorithm that is specially trained to measure changes of abnormalities in images. Such algorithm facilitates assessment of series of images that are indicative of clinically actionable changes. It should be noted that this decision algorithm should preferably be invoked automatically before a clinician reviews the case, especially in the case of triage.

The threshold may be a value (e.g. a volume, measurement) or a predefined region that should not be exceeded. However, the threshold may also be a limit for the measure of the change itself, e.g. a threshold for the first derivative of the change with respect to time.

In the case the assessed changes exceed the predefined threshold:
- triggering an alert notification and/or change a worklist prioritization concerning the respective patient.

Thus, the change exceeding the threshold indicates that there is a dangerous condition and medical staff could be alerted. On the other hand, since there may be other patients within a dangerous condition, the respective patient may be prioritized on a worklist. This prioritization is preferably depending on the difference between the threshold and the change (about how much the change exceeds the threshold) and/or the change itself (when exceeding the threshold, a big change may be prioritized over a small change).

Optionally, in the case the assessed changes do not exceed the predefined threshold, this may be recorded for further decisions.

A determination system according to the invention for automatically determining (alertable) changes of a condition of a patient, and especially designed for performing the method according to the invention, comprises the following components:
- A data interface designed for receiving at least a first image and a second image, both showing the same predefined anatomical region of the patient, wherein the images have been recorded during different examinations of the patient,
- an examination unit designed for examining the images for abnormalities of a predetermined type,
- a determination unit designed for
   i) determining changes of the abnormality between the first and the second image,
   ii) assessing the determined changes,
   iii) determining whether the assessed changes exceed a predefined threshold by using a decision algorithm,
- an output unit designed for triggering an alert notification and/or change a worklist prioritization concerning the respective patient in the case the assessed changes exceed the predefined threshold.

It is particularly preferred that the determination unit comprises an (especially deep) learning algorithm fashioned in such way as to facilitate the assessment of changes, e.g. in a pneumothorax. In broad strokes, the algorithm performs a size and location analysis (e.g. of the pneumothorax) on the first image and the second image (e.g. a follow-up scan and a prior scan) and if there is a severe change (e.g. the pneumothorax has grown or developing in a new anatomical location), the case is flagged as urgent.

A client device according to the invention is designed to communicate with a determination system according to the invention and send a first image and a second image to this determination system and receive data from the determination system. It is preferred that the client device is further designed to process a request of the determination system for acquiring an image, searching a corresponding image in a database and sending this image as a second image to the determination system. In short: a client device is designed to work together with the method according to the invention.

Since medical data is sent between determination system and client device, especially if the data processing is accomplished in a cloud (outside of hospital premises), care should be taken that confidential data is hidden from third parties, e.g. by encryption or routines such as de-identification / re-identification.

A control device according to the invention for controlling a medical imaging system comprises a determination system according to the invention. Alternatively or additionally it is designed to perform the method according to the invention. The control device may comprise additional units or devices for controlling components of the medical imaging system.

A medical imaging system, especially a radiographic system, a computer tomography system or a magnetic resonance system, comprising a control device according to the invention.

Some units or modules of the determination system mentioned above can be completely or partially realized as software modules running on a processor of a computing system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the inventive method when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

A preferred embodiment of the method comprises the following steps:
- automatically segmenting the images, wherein at least the region of interest is segmented, preferably using a machine learning algorithm, wherein an abnormality is made an individual segment (this step may be performed during examining the images),
- preferably: registering the segmented images,
- comparing areas of an abnormality in the images by determining the change of a segmented abnormality in the first image in respect to a corresponding segmented abnormality or a respective area in the second image (determining changes) in form of a numerical value (assessing the changes).

It is preferred that the current image (e.g. of a follow-up) is processed as first image and a prior image (as second image) is processed in the case an abnormality, e.g. a pathology, is found in the current image. The method especially extends to images recorded at different physical locations. For example, the patient can be shifted from a nursing home to the hospital. The prior image could be a portable CXR-scan and the next image could be made in an Intensive Care Unit.

Thus, only if an abnormality is found in the first image, a second image is received and examined. According to a preferred embodiment, the second image is determined with a routine comprising the following steps:
a) Providing a patient identifier designed to identify the patient from whom the first image was taken, preferably by reading patient information from the data of the first image, especially from a DICOM header.
b) Searching for a second image of the patient in a database or trigger a recording of a second image, wherein the second image shows the predefined anatomical region based on the patient identifier and information about the predefined anatomical region, preferably by querying an image archive, especially PACS, whether an image of a prior scan of same patient in a given time frame is available.
   By knowing the patient from the patient identifier, other images can be searched. Only images are selected that show the predefined anatomical region and that are preferably recorded in a predefined time frame, e.g. not more than a day, week or month prior the recording of the first image.
c) If there is an image found, using this image as second image for the method.

Thus, the first and second image are here not received at the same time, but the steps receiving and examining the images of the method are distributed in:
- receiving a first image, showing a predefined anatomical region of the patient,
- examining the first image for abnormalities of a predetermined type,
and in the case an abnormality is found in the first image:
- sending a request to search for a second image (preferably together with the patient identifier),
- receiving a second image, showing also the predefined anatomical region of the patient, wherein the images have been recorded during different examinations of the patient,
- examining the second image for abnormalities of a predetermined type.

Since an abnormality is at least found in the first image, the method proceeds with the determination of changes.

It is preferred to use a machine learning algorithm, especially a deep learning algorithm, for examining (and especially also segmenting) the images.

For example, a preferred method comprises the following steps:
- acquire a first image, e.g. an X-ray image
- optionally: send the first image to a determination system, especially via DICOM communication,
- optionally: pre-processing of the first image, especially normalization or de-noising,
- segmentation of the first image (e.g. of the lung area), especially using a (deep) machine learning algorithm,
- examining the first image for abnormalities (e.g. a pneumothorax), especially using a (deep) machine learning algorithm, and in the case an abnormality is found
   a) read patient identifier, e.g. from a DICOM header of the first image,
   b) query an image archive (such as e.g. PACS) whether a prior image of the same patient in a given time frame (e.g. same day, same week, etc.) is available,
   c) if yes, fetch this prior image as second image,
- optionally: send the second image to the determination system, especially via DICOM communication,
- optionally: pre-processing of the second image, especially normalization or de-noising,
- segmentation of the second image (the same area as segmented in the first image), especially using a (deep) machine learning algorithm, preferably using the same algorithm as for the first image,
- examining the second image for abnormalities (the same as for the first image), especially using a (deep) machine learning algorithm,
- optional: registering both images,
- compare abnormalities of the first image with corresponding abnormalities or sections (if there is no abnormality) of the second image, e.g. a pneumothorax relative to the ratio of the lung volume,
- determining a value for the change of the abnormality between the images (assessment),
- in the case the change exceeds a predefined threshold, e.g. if the percentage affected area on the first image is bigger (by a given offset) than in the second image, flag the images of the patient and prioritize the patient in a worklist.

According to a preferred embodiment, before processing an image, this image is pre-processed and/or after processing an image, this image is post-processed, and/or after comparing the images, the values of the changes are post-processed. Concerning processing-techniques, it is preferred that the pre-processing and/or the post processing is based on means selected from the group comprising normalization, de-noising, image-reconstruction, processing with artificial object determination, smoothing, edge enhancement, field segmentation (e.g. lung field-segmentation), bone removal, spectral energy material decomposition, landmark detection, image normalization (such as flavor homogenization), view correction (e.g. auto rotating images as portables can be taken with arbitrary geometries), checking for image quality (such as exposure or inspiration level), removing of confounding lines and tubes.

According to a preferred embodiment, in the course of automatically segmenting the images, the thorax is segmented, preferably the lung or the thoracic cavity, and if existing an abnormality in the thorax is segmented, preferably a pneumothorax. It is preferred that in the course of comparing the images (determining changes and assessing the changes), the size of a pneumothorax is assessed by measuring the sizes following American guidelines or British guidelines. It is also preferred that the comparison of the images is followed or preceded by a normalization relative to the ratio of the lung volume or size.

According to a preferred embodiment, a machine learning algorithm is used for examining the images and/or for determining changes of an abnormality and/or for determining whether the assessed changes exceed a predefined threshold. This machine learning algorithm is preferably a deep-learning network, preferably a deep belief network, ResNet, DenseNet, Autoencoder, capsule network, generative adversarial network, Siamese network, convolutional neural network, deep reinforcement learning algorithm, or preferably based on a machine learning technique of the group support vector machine, Bayesian model, decision tree and k-means clustering.

The training methodology of such machine learning algorithm is preferably based on the following steps:
1. Curation of a training-dataset, especially a multi-center, multi-vendor anonymized dataset.
2. Curation of mimickers for contrastive learning such as datasets with bullae, curvilinear shadows produced by: skin folds, hair, companion shadows, clothing, tubes and lines.
3. Data harmonization to minimize inter-site variations.
4. Quality Control with expert medical professions, especially with a check for exposure, inspiration, field of view, image quality etc.
5. Annotation by multiple independent experts, especially comprising image level labelling, zone-level labelling, freehand contouring, characterization of the abnormality (e.g. a pneumothorax "PTX") like "tension", "apical", "basilar".
6. Annotation of labelling confounders, e.g. view position (PA, AP, Portable, Supine, Lateral etc.), presence of chest tube (drainage tubes), other lines such as CVC catheters.
7. Annotation of standardized measurements, such as the apex-to-cupola distance, segmentation of lung fields, or of the thoracic cavity.
8. Aggregation of annotations across multiple annotators.
9. Training of the machine learning algorithm to predict the presence of an abnormality (e.g. a PTX) in a single image, preferably with an architecture selected form the group comprising a deep-learning network, preferably a deep belief network, ResNet, DenseNet, Autoencoder, capsule network, generative adversarial network, Siamese network, convolutional neural network, deep reinforcement learning algorithm, or preferably based on a machine learning technique of the group support vector machine, Bayesian model, decision tree and k-means clustering.

In a testing scenario, the following steps are preferred:
1. Segmenting suspected regions of an abnormality (e.g. PTX regions) in the images.
2. estimating extent with respect to a surrounding area (e.g. the lung-area).
3. estimating dimensions of the abnormalities (e.g. the Apex-to-cupola distance).
4. performing differential analyses to see if new regions of interest are suspected (the change).
5. check whether the change of the abnormality exceeds a predefined threshold (e.g. whether a PTX in the latest time-point is tension in nature).
6. If yes: send an alert or prioritize this patient for the next examination.
   The last item can comprise multiple further queries, e.g: Check if the abnormality (e.g. the PTX size) has grown.
   If yes: alert.
   If no: check if the abnormality (e.g. the PTX) is present in new areas.
   If yes: alert.
   If no: place case in a significance list.
7. If the size of the abnormality (e.g. the PTX size) is shrinking, preferably mark the case, but do not alert.
8. If the abnormality (e.g. the PTX) is resolved, preferably do not mark the case and do not alert.

According to a preferred embodiment, the images are digital images, preferably acquired with an X-ray-system, a computer tomography-system, a magnetic resonance imaging system, optical coherence tomography, photoacoustic tomography, positron emission tomography, Single-photon emission computed tomography, diffuse scattering tomography, infrared tomography, ultrawide bandwidth tomography or ultrasound.

According to a preferred embodiment, an abnormality is a (radiographic) finding of the group comprising malpositioned lines or tubes of immediate clinical concern, tension pneumothoraces, pulmonary emboli, lung lesions with high possibility of being active tuberculosis, superior vena cava occlusion, large pericardial effusion and/or suspected tamponade of any cause, active post-traumatic hemorrhage, tracheal obstruction, lobar or lung collapse, pneumomediastinum, interstitial emphysema, extensive subcutaneous emphysema, pleural effusion, significant vena cava compression and pneumonia, or a non-thoracic finding from the group comprising suspected nonaccidental trauma, malpositioned line or tube of immediate clinical concern (e.g., ET tube or enteric tube in bronchus), allergic reaction or other adverse event, foreign body with potential immediate and/or severe consequences, intracranial or spinal hemorrhage (parenchymal, subarachnoid, subdural, epidural), non-hemorrhagic stroke or suspected stroke, thrombolytic candidate, intracranial mass with significant mass effect (midline shift/herniation/hydrocephalus), brain herniation, symptomatic hydrocephalus (malfunctioning shunt or new diagnosis of any cause), depressed skull fracture, post-traumatic pneumocephalus, arterial dissection, brain death (nuclear study or other), severe spinal cord compression of any cause, unstable spine fracture, cord hemorrhage or infarct, airway obstruction, unexplained pneumoperitoneum, closed loop intestinal obstruction, intestinal ischemia and/or portal/mesenteric, venous gas, pseudoaneurysm or active hemorrhage (post-trauma, GI bleed, other), high-grade intra-abdominal organ injury, testicular torsion, ovarian torsion, ectopic pregnancy (high likelihood), placental abruption, uterine rupture, high grade kidney injury and/or ureteral or bladder injury post-trauma, non-spinal fracture and/or dislocation with risk of vascular compromise, necrotizing fasciitis, Ruptured/leaking arterial aneurysm (thoracic or abdominal aortic or other), limb-threatening arterial or venous occlusion or high-grade stenosis, arterial dissection and intramural hematoma (aortic or other).

According to a preferred embodiment, in the course of comparing the areas of an abnormality in the images, the speed of growth is determined, preferably based on the time difference between the recording of images, especially by determining the difference of the area between the images relative to the time between the recording of the images. In particular, the speed of growth is the speed of growth of the areas of the abnormality in the image. This time dependence (e.g. a slope of a curve) has the advantage that such dependence (e.g. the slope) provides a measure about how fast the change occurred.

In general, the images may provide information about the time point of their recording and the difference between the time of recording of the first image and the second image is preferably calculated together with the changes. The changes are then preferably compared with the threshold based on this time difference, so that e.g. a faster growth exceeds the threshold wherein a slower growth of the same size doesn't.

According to a preferred embodiment, the method is performed in that a client device (according to the invention) sending the images and a main computer-system (with the determination system according to the invention) that is capable of performing the method according to the invention receives and processes the images. The images are preferably DICOM-datasets. The main computing system may be a computing system of a medical imaging apparatus (according to the invention), a computing system of an edge device of a network or a computing system of a medical institute (on-premise processing). The determination system could also be designed as a dedicated piece of hardware that can be connected to a medical imaging device, e.g. a CT scanner.

Preferred technical components for the invention are: an image viewer such as PACS, a worklist (e.g. PACS- or RIS-driven), AI software (software for a machine learning algorithm) to perform computation, computational hardware for AI software (either cloud-based or on-premise).

According to a preferred determination system, components of the determination system are part of a data-network, wherein preferably the data-network and a medical imaging system (e.g. a CT-system which provides image data) are in data-communication with each other, wherein the data-network preferably comprises parts of the internet and/or a cloud-based computing system, wherein preferably the determination system according to the invention or a number of components of this determination system is realized in this cloud-based computing system. For example, the components of the system are part of a data-network, wherein preferably the data-network and a medical imaging system which provides the image data are in communication with each other. Such a networked solution could be implemented via an internet platform and/or in a cloud-based computing system.

The method may also include elements of "cloud computing". In the technical field of "cloud computing", an IT infrastructure is provided over a data-network, e.g. a storage space or processing power and/or application software. The communication between the user and the "cloud" is achieved by means of data interfaces and/or data transmission protocols.

In the context of "cloud computing", in a preferred embodiment of the method according to the invention, provision of data via a data channel (for example a data-network) to a "cloud" takes place. This "cloud" includes a (remote) computing system, e.g. a computer cluster that typically does not include the user's local machine. This cloud can be made available in particular by the medical facility, which also provides the medical imaging systems. In particular, the image acquisition data is sent to a (remote) computer system (the "cloud") via a RIS (Radiology Information System) or a PACS (Picture Archiving and Communication System).

Within the scope of a preferred embodiment of the system according to the invention, the abovementioned units (examination unit, determination unit, output unit) are present on the "cloud" side. A preferred system further comprises, a local computing unit (client device) connected to the determination system via a data channel (e.g. a data-network, particularly configured as RIS or PACS).

It should be noted that the images could be 2D-images or 3D-images. By using deep learning algorithms, the images do not have to be reconstructed images, but could also comprise raw data. In general, it is preferred that the images are DICOM-datasets.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a simplified CT system according to an embodiment of the invention.
Figure 2 shows a block diagram of the process flow of a preferred method according to the invention.
Figure 3 depicts the American guideline and the British guideline.
Figure 4 shows a block diagram of a process flow of a further preferred method according to the invention.
Figure 5 shows a diagram of a process flow of a further preferred method according to the invention.

In the diagrams, like numbers refer to like objects throughout. Objects in the diagrams are not necessarily drawn to scale.

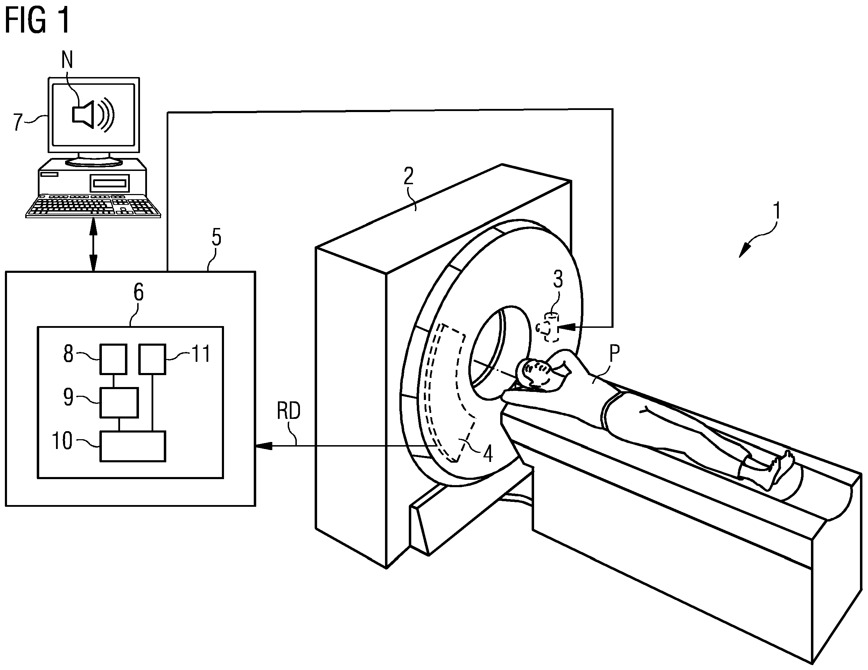

Figure 1 shows a simplified computer tomography system 1 with a control device 5 comprising a determination system 6 for carrying out the method according to the invention. The computer tomography system 1 has in the usual way a scanner 2 with a gantry, in which an x-ray source 3 with a detector 4 rotates around a patient and records raw data RD that is later reconstructed to images by the control device 5.

It is pointed out that the exemplary embodiment according to this figure is only an example of an imaging system and the invention can also be used on theoretically any imaging system that is used in medical and non-medical environment. Likewise, only those components are shown that are essential for explaining the invention. In principle, such imaging systems and associated control devices are known to the person skilled in the art and therefore do not need to be explained in detail.

The imaging system (here the CT system 1) records images I1, 12 that are processed by the determination system 6 according to the invention. The determination system 6 comprises a data interface 8, an examination unit 9 and a determination unit 10 to perform the steps of the method according to the invention.

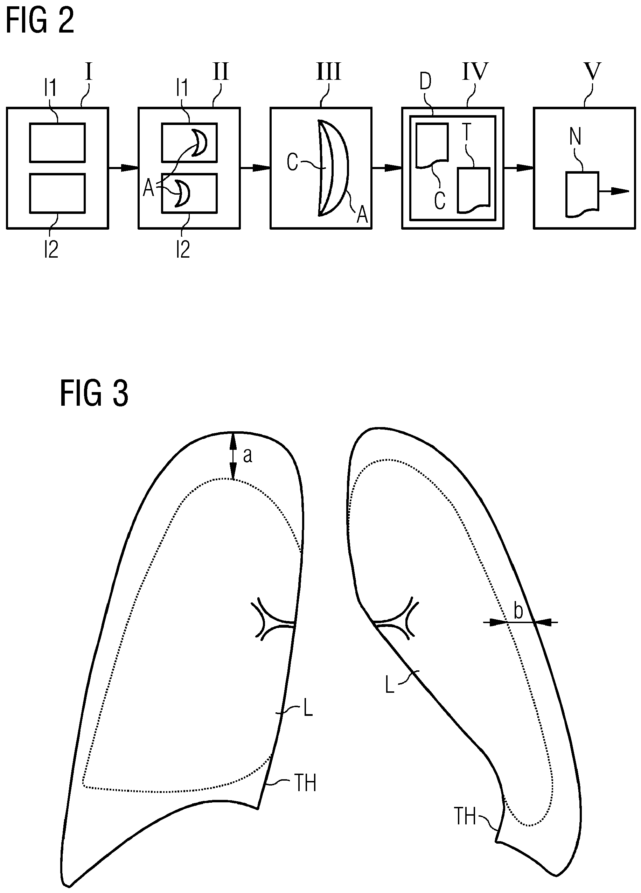

The data interface 8 is designed for receiving images I1, I2 (see e.g. figure 2 or 3) both showing the same predefined anatomical region TH, e.g. the thorax TH (see e.g. figure 3) of the patient P. The examination unit 9 is designed for examining the received images I1, I2 for abnormalities A of a predetermined type. The determination unit 10 is designed for different purposes of the method.

First, the determination unit 10 is designed for determining changes C of an abnormality A between the first image I1 and the second image 12. Then, the determination unit 10 is designed for assessing the determined changes C and furthermore, the determination unit 10 is designed for determining whether the assessed changes C exceed a predefined threshold T by using a decision algorithm D.

The output unit 11 is designed for triggering an alert notification N and/or to change a worklist prioritization concerning the respective patient P in the case the assessed changes C exceed the predefined threshold T.

For communicating with the determination system 6, a user can use the terminal 7 that is here designed as a client device 7 according to the invention. The client device 7 is here designed to communicate with the determination system 6 and to send images to this determination system 6 and receive data from the determination system 6, here an alert notification N. The client device 7 may here also be designed to process a request of the determination system 6 for acquiring an image, searching a corresponding image in a database and sending the image as a second image I2 to the determination system 6. Figure 2 shows a block diagram of the (basic) process flow of a preferred method according to the invention for automatically determining changes C of a condition of a patient P.

In Step I, at least a first image I1 and a second image I2 are received, both showing the same predefined anatomical region TH, e.g. the Thorax TH (see figure 3) of the patient P, wherein the images I1, I2 have been recorded during different examinations of the patient P.

In Step II, the images I1, I2 are examined for abnormalities A of a predetermined type.

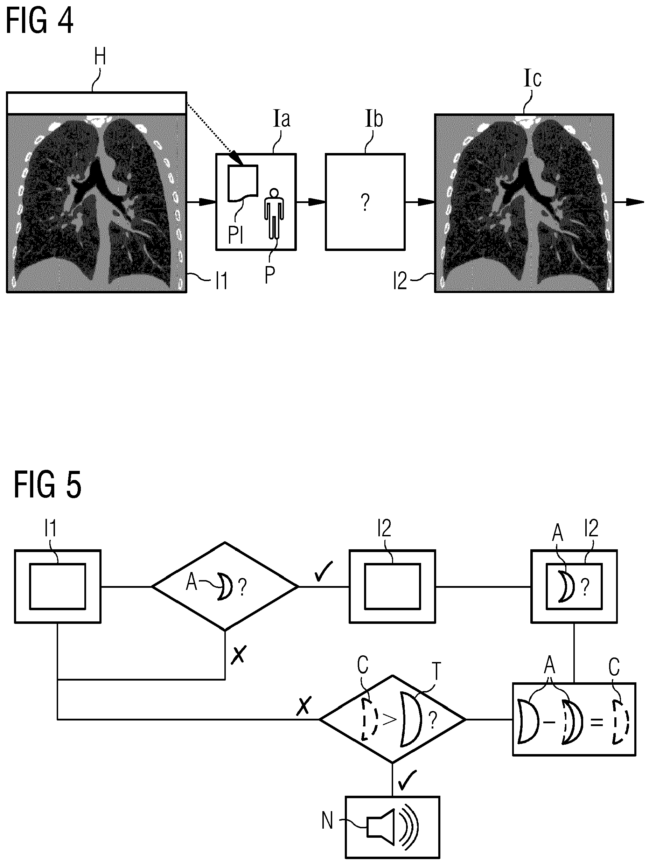

It should be noted that the images I1, I2 do not need to be received or examined together, as shown, e.g. in figures 4 or 5, the first image I1 can be received and examined and only in the case an abnormality is found in the first image I1, a second image I2 is requested, received and examined.

If no abnormality is found, the method does not need to proceed. It could wait for receiving another number of images.

In the case an abnormality A is found in at least one of the images I1, 12, the method proceeds with the following steps:
In step III, changes C of an abnormality A is determined between the first image I1 and the second image I2 and since this should be done with a determination system 6 as shown in figure 1, the change is already present as a digital dataset and, therefore, already assessed.

In step IV, it is determined whether the assessed changes C exceed a predefined threshold T. This is done by using a decision algorithm D, especially a trained machine learning algorithm.

In the case the changes C do not exceed the threshold, there must be done nothing. However, it would be advantageous to record this issue for later examinations.

In the case the assessed changes C exceed the predefined threshold T, the method proceeds with step V: triggering an alert notification N. Alternatively or additionally, a worklist prioritization concerning the respective patient P could be changed, e.g. such that the patient is prioritized or not depending on the value of the change C.

Figure 3 depicts the American guideline (a left) and the British guideline (b right) for evaluating a pneumothorax of the lung L in the thorax TH. Changes of the values a or b could be regarded as a very simple example for an assessed change. According to the American guideline, the distance a from the apex to the cupola is measured. According to the British guideline, the interpleural distance b at level of the hilum is measured.

Figure 4 shows a block diagram of a process flow of a further preferred method according to the invention, wherein the second image I2 is determined with a routine comprising the following steps:
There is already a first image I1 received in the course of step I of a method as shown in figure 2. This first image I1 could be a DICOM-file with a header H comprising information about the patient P and the preferences concerning the recording of this first image I1.

In Step Ia, a patient identifier PI is provided, especially by parsing the header H for patient related information. With this patient identifier PI it is possible to identify the patient P from whom the first image I1 was taken from.

In step Ib, a search query is sent for searching a second image I2 of the patient P in a database. The search is performed such that the second image I2 must show the predefined anatomical region TH shown in the first image I1. This could be achieved by using the patient identifier PI and information about the predefined anatomical region TH. For example, an image archive such as PACS could be queried, whether an image of a prior scan of same patient P in a given time frame is available.

In step Ic, the found image is used as second image I2 for the method as shown in figures 1 or 5.

Figure 5 shows a block diagram of a process flow of a preferred method that combines the methods shown in figures 2 and 3. The example deals with images of the lung area and a search for a pneumothorax, but can be extrapolated to fit other applications, as well. Concerning the components mentioned in the following, figure 1 could be consulted.

First, a user sends a first image I1 from a client device 7 to a determination system 6.

Then the first image I1 is examined for an abnormality A. If there is no abnormality A found, the method returns to the first step, if there is an abnormality found, the method proceeds with the next step, where a second image I2 is provided using e.g. a method as shown in figure 3. In addition, further images recorded at different points of time could also be provided as well as additional information, e.g. what was the result, when the patient P was examined last time by this method.

The second image is then examined for the same abnormality A. Regarding step III of the method shown in figure 2, there is always an abnormality A found at this stage, since a second image I2 is only processed, if there is an abnormality A found in the first image I1. Thus, the algorithm proceeds to the next stage.

Now, the determination of changes C begins. Since the abnormalities A in the images I1, I2 are well known, changes could be determined between the abnormalities A shown in the first image I1 and the second image I2 by comparing the abnormalities A. If there is no abnormality A in the second image 12, the change is big (from zero to the size of the abnormality A). As already indicated above, since the client device 7 and the determination system 6 are computing units, the changes C are automatically assessed, i.e. being present in as numerical values, fields or as functions.

Now it is determined, whether the assessed changes C exceed a predefined threshold T (second rhomb). This is done by using a decision algorithm D, e.g. a deep learning algorithm trained for this special purpose. If the changes C do not exceed the threshold T (as shown here, the threshold T is bigger), the method returns to the first step. If the changes C exceed the threshold T, then there is a certain danger for the patient P or respectively an actionable change that is complying with Standard of Care.

In this positive case, an alert notification N is triggered and/or a worklist prioritization concerning the respective patient P is changed.

Nearly all stages shown in figure 5 could be accomplished with the use of a (trained) KI. Examining the images I1, I2 for abnormalities A could be done by automatically segmenting the images I1, 12, wherein at least the predefined anatomical region TH is segmented by using a machine learning algorithm, wherein an abnormality A is made an individual segment.

Also, the comparison of the abnormality A in the images I1, I2 and determining the changes C of a (segmented) abnormality could by performed by a trained KI as well as the comparison of the change C with the threshold.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The mention of a "unit" or a "device" does not preclude the use of more than one unit or device.

## Claims

1. A method for automatically determining changes (C) of a condition of a patient (P), comprising the steps:
- receiving at least a first image (I1) and a second image (12), both showing the same predefined anatomical region (TH) of the patient (P), wherein the images (I1, 12) have been recorded during different examinations of the patient (P),
- examining the images (I1, 12) for abnormalities (A) of a predetermined type, and in the case an abnormality (A) is found in at least one of the images (I1, 12):
- determining changes (C) of an abnormality (A) between the first image (I1) and the second image (12),
- assessing the determined changes (C),
- determining whether the assessed changes (C) exceed a predefined threshold (T) by using a decision algorithm (D),
- in the case the assessed changes (C) exceed the predefined threshold (T): triggering an alert notification (N) and/or change a worklist prioritization concerning the respective patient (P).

2. The method according to claim 1, comprising the steps:
- automatically segmenting the images (I1, 12), wherein at least the anatomical region (TH) is segmented, preferably using a machine learning algorithm, wherein an abnormality (A) is made an individual segment,
- preferably: registering the segmented images (I1, 12),
- comparing areas of an abnormality (A) in the images (I1, 12) by determining the changes (C) of a segmented abnormality (A) in the first image (I1) in respect to a corresponding segmented abnormality (A) or a respective area in the second image (12) in form of a numerical value.

3. The method according to one of the preceding claims, wherein the second image (12) is determined with a routine comprising the following steps:
a) providing a patient identifier (PI) designed to identify the patient (P) from whom the first image (I1) was taken, preferably by reading patient information from the data of the first image (I1), especially from a DICOM header (H),
b) searching for a second image (12) of the patient (P) in a database or trigger a recording of a second image (12), wherein the second image (12) shows the predefined anatomical region (TH) based on the patient identifier (PI) and information about the predefined anatomical region (TH), preferably by querying an image archive, especially PACS, whether an image of a prior scan of same patient (P) in a given time frame is available,
c) if there is an image found, using this image as second image (12) for the method.

4. The method according to one of the preceding claims, wherein before processing an image (I1, 12), this image (I1, 12) is pre-processed and/or after processing an image (I1, 12), this image is post-processed, and/or after comparing the images (I1, 12), the values of the changes (C) are post-processed,
wherein the pre-processing and/or the post processing is preferably based on means selected from the group comprising normalization, de-noising, image-reconstruction, processing with artificial object determination, smoothing, edge enhancement, field segmentation, bone removal, spectral energy material decomposition, landmark detection, image normalization, view correction, checking for image quality, removal of confounding lines and tubes.

5. The method according to one of the preceding claims, wherein the images (I1, 12) are automatically segmented and in the course of automatically segmenting the images (I1, 12), the thorax (TH) is segmented, preferably the lung (L) or the thoracic cavity, and if existing an abnormality (A) in the thorax (TH) is segmented, preferably a pneumothorax, preferably wherein in the course of comparing the images, the size of a pneumothorax is assessed by measuring the sizes following American guidelines or British guidelines, preferably wherein the comparison of the images (I1, 12) is followed or preceded by a normalization relative to the ratio of the lung volume.

6. The method according to one of the preceding claims, wherein a machine learning algorithm is used for examining the images (I1, 12) and/or for determining changes (C) of an abnormality (A) and/or for determining whether the assessed changes (C) exceed a predefined threshold (T), wherein the machine learning algorithm is a deep-learning network, preferably a deep belief network, ResNet, DenseNet, Autoencoder, capsule network, generative adversarial network, Siamese network, convolutional neural network, deep reinforcement learning algorithm, or preferably based on a machine learning technique of the group support vector machine, Bayesian model, decision tree and k-means clustering.

7. The method according to one of the preceding claims, wherein the images (I1, 12) are digital images, preferably acquired with an X-ray-system, a computer tomography-system, a magnetic resonance imaging system, optical coherence tomography, photoacoustic tomography, positron emission tomography, Single-photon emission computed tomography, diffuse scattering tomography, infrared tomography, ultrawide bandwidth tomography or ultrasound.

8. The method according to one of the preceding claims, wherein an abnormality (A) is a finding of the group comprising malpositioned lines or tubes of immediate clinical concern, tension pneumothoraces, pulmonary emboli, lung lesions with high possibility of being active tuberculosis, superior vena cava occlusion, large pericardial effusion and/or suspected tamponade of any cause, active post-traumatic hemorrhage, tracheal obstruction, lobar or lung collapse, pneumomediastinum, interstitial emphysema, extensive subcutaneous emphysema, pleural effusion, significant vena cava compression and pneumonia,
or a non-thoracic finding from the group comprising suspected nonaccidental trauma, malpositioned line or tube of immediate clinical concern, allergic reaction or other adverse event, foreign body with potential immediate and/or severe consequences, intracranial or spinal hemorrhage (parenchymal, subarachnoid, subdural, epidural), non-hemorrhagic stroke or suspected stroke, thrombolytic candidate, intracranial mass with significant mass effect, brain herniation, symptomatic hydrocephalus, depressed skull fracture, post-traumatic pneumocephalus, arterial dissection, brain death, severe spinal cord compression of any cause, unstable spine fracture, cord hemorrhage or infarct, airway obstruction, unexplained pneumoperitoneum, closed loop intestinal obstruction, intestinal ischemia and/or portal/mesenteric, venous gas, pseudoaneurysm or active hemorrhage, high-grade intra-abdominal organ injury, testicular torsion, ovarian torsion, ectopic pregnancy, placental abruption, uterine rupture, high grade kidney injury and/or ureteral or bladder injury post-trauma, non-spinal fracture and/or dislocation with risk of vascular compromise, necrotizing fasciitis, ruptured/leaking arterial aneurysm, limb-threatening arterial or venous occlusion or high-grade stenosis, arterial dissection and intramural hematoma.

9. The method according to one of the preceding claims, wherein in the course of comparing the areas of an abnormality (A) in the images (I1, 12), the speed of growth is determined, preferably based on the time difference between the recording of images, especially by determining the difference of the area between the images relative to the time between the recording of the images (I1, 12).

10. A determination system (6) for automatically determining changes (C) of a condition of a patient (P), especially designed for performing the method according to any of the preceding claims, comprising:
- a data interface (8) designed for receiving at least a first image (I1) and a second image (12), both showing the same predefined anatomical region (TH) of the patient (P), wherein the images (I1, 12) have been recorded during different examinations of the patient (P),
- an examination unit (9) designed for examining the images (I1, 12) for abnormalities (A) of a predetermined type,
- a determination unit (10) designed for
i) determining changes (C) of an abnormality (A) between the first image (I1) and the second image (12),
ii) assessing the determined changes (C),
iii) determining whether the assessed changes (C) exceed a predefined threshold (T) by using a decision algorithm (D),
- an output unit (11) designed for triggering an alert notification (N) and/or change a worklist prioritization concerning the respective patient (P) in the case the assessed changes (C) exceed the predefined threshold (T).

11. The determination system (6) according to claim 10, being designed as computing system of a medical imaging apparatus (1), a computing system of an on-edge device, a computing system of a medical institute, as a cloud system or as a dedicated piece of hardware that is designed to be connected to a medical imaging device (1).

12. A client device (7) designed to communicate with a determination system (6) according to claim 10 or 11 and send a first image (I1) and a second image (12), to this determination system (6) and receive data from the determination system (6),
preferably wherein the client device (7) is designed to processing a request of the determination system (6) for acquiring an image, searching a corresponding image in a database and sending this image as a second image (12) to the determination system (6).

13. A method comprising the steps:
- sending at least a first image (I1) and a second image (12) from a client device (7) according to claim 12 to a determination system (6) according to claim 10 or 11,
- processing the images (I1, 12) with the determination system (6) by applying the method according to one of claims 1 to 9,
- in the case the assessed changes (C) exceed the predefined threshold (T), sending an alert notification (N) and/or changes for a worklist prioritization concerning the respective patient (P) to the client system (7).

14. A computer program product comprising a computer program that is directly loadable into a computing system, which comprises program elements for performing steps of the method according to any of claims 1 to 9 or 13 when the computer program is executed by the computing system.

15. A computer-readable medium on which is stored program elements that can be read and executed by a computer unit in order to perform steps of the method according to any of claims 1 to 9 or 13 when the program elements are executed by the computer unit.
